(19) Europäisches Patentamt
European Patent Office
Office européen des brevets

(11) Publication number: **0 275 998 A2**

(12) # EUROPEAN PATENT APPLICATION

(21) Application number: 88100800.7

(22) Date of filing: 20.01.88

(51) Int. Cl.[4]: A 61 K 31/13

(30) Priority: 22.01.87 IL 81361

(43) Date of publication of application: 27.07.88 Bulletin 88/30

(84) Designated Contracting States: AT BE CH DE FR GB IT LI NL

(71) Applicant: YISSUM RESEARCH DEVELOPMENT COMPANY OF THE HEBREW UNIVERSITY OF JERUSALEM
46 Jabotinsky Street
Jerusalem, 92 182 (IL)

(72) Inventor: Ilani, Asher
12 Metudella St.
Jerusalem (IL)

Lichtstein, David
2/2 Ramat Sharet
Jerusalem (IL)

Somer, Haim
37 Shahal St.
Jerusalem (IL)

Deutsch, Joseph
21 Hameshoreret Rachel St.
Jerusalem (IL)

Goitein, Kalman
10 Ussishkin St.
Jerusalem (IL)

Bacaner, Marvin
4401 Fremont Avenue
South Minneapolis, MI 55409 (US)

(74) Representative: Huber, Bernhard, Dipl.-Chem. et al
Möhlstrasse 22 Postfach 860 820
D-8000 München 86 (DE)

(54) **Pharmaceutical preparation for treatment of hyperacidity in body fluids.**

(57) Pharmaceutical preparations for the treatment of hyperacidity in body fluids comprising as active ingredient a compound of the formula:

$$R^3-CH_2(CHOH)_3-\overset{R^2}{\overset{|}{C}}H-CH_2R^1 \qquad (I)$$

wherein each of $R^1$, $R^2$ and $R^3$ represents a hydroxyl group or an amino group optionally substituted by lower alkyl, at least one of $R^1$, $R^2$ and $R^3$ being said amino group, optionally substituted by lower alkyl, provided that $R^2$ and $R^1$ or $R^2$ and $R^3$ cannot simultaneously represent an amino group; or enantiomers or diastereoisomers thereof; or oligomers thereof: and pharmaceutically acceptable salts thereof, optionally comprising pharmaceutically acceptable additives, in a suitable carrier or diluent.

The pharmaceutical preparations can be used for treatment of gastric hyperacidity and for treatment of hyperacedemia.

EP 0 275 998 A2

**Description**

PHARMACEUTICAL PREPARATION FOR TREATMENT OF HYPERACIDITY IN BODY FLUIDS

The present invention relates to pharmaceutical preparations for the treatment of acidosis and gastric hyperacidity and the medical preparations for emergency treatment of blood loss.

More particularly the invention relates to pharmaceutical preparations for the treatment of acidosis and gastric hyperacidity and blood loss conditions comprising as active ingredient amino (optionally substituted) - reduced sugar derivatives optionally further comprising pharmaceutically acceptable additives in a pharmaceutically acceptable carrier or diluent.

BACKGROUND OF THE INVENTION

Acidosis is defined as a condition of abnormally high level of acidity in the body fluids. Metabolic acidosis may arise from three main causes:

(a) An increased formation of acids during the metabolic processes. Such increase occurs, for example, in diabetes or starvation or as a result of reduced levels of oxygen in the tissues, in emergency conditions as, for example, heart arrest or severe accidents:

(b) Diminished excretion of hydrogen ions as, for example, in situations like kidney failure:

(c) Increased loss of base such as bicarbonate ions, as occurs in diarrhea.

The increased concentration of hydrogen ions in the plasma causes severe damage to tissues and cells and in extreme situations those changes may be irreversible. Therefore, these conditions require prompt treatment with antiacidotic agents.

These conditions of acidosis have been and are traditionally treated by intravenous administration of sodium bircarbonate solutions. Sodium bicarbonate is used quite lavishly in emergency conditions and intenseive care units (1).

However, the use of sodium bicarbonate has several disadvantages. This treatment increases plasma osmolality (2) and partial pressure of carbon dioxide in plasma (3). The increase in osmolality may cause various complications, decreasing chances of survival. The rise in carbon dioxide pressure impairs myocardial contractility, and also produces undesirable neurological effects (3,4).

Furthermore, it is well established today that an increase in sodium ions may be the primary cause of essential hypertension and therefore its abundant use in diet and pharmaceutical preparations should be avoided (5).

The literature also describes experiments of combating acidosis with tris(hydroxymethyl)-aminomethane buffer mixtures (6). While tris buffer mixtures may be more effective than traditional sodium bicarbonate in correcting the acid-base disturbance, its clinical use, if any, is limited mainly because of its side effects such as, inter alia, thrombophlebitic changes or serous tissue irritation (7).

As stated above the present invention also relates to pharmaceutical preparations for the treatment of conditions of gastric hyperacidity. The stomach secretes concentrated acid into its lumen as a part of the normal digesting process. It is well known that a pathological increase in the acid secretion induces reflux esophagitis and peptic ulcers. These diseases are currently treated by antacid preparations comprising as active ingredient aluminum hydroxide, magnesium hydroxide, calcium carbonate or sodium bicarbonate (reference is made to a review on gastric antacid agents (8) ).

One of the common problems of using these preparations is the continued hypersecretion of HCl after the intragastric pH has returned to normal (acid rebound). The main mechanism for this effect is the non-pH-related effect of the products (salts: $MgCl_2$, $AlCl_3$, $CaCl_2$) to stimulate gastrin secretion and hence production of acid (9).

Another aspect of the invention is to provide intravenous infusion solutions in cases of blood loss. Immediate blood transfusion to patients suffering haemorrhage is sometimes impossible, for example, under field conditions, or due to temporary shortage of blood units in cases of catastrophes or very rare blood group of the patient. In other cases there may be a necessity or desire to delay or totally avoid blood transfusion. At present, in such emergency conditions patients usually first receive infusion of plasma or physiological solutions, and blood is transfused at later stages. Blood transfusion cannot be delayed for very long periods of time and almost always cannot be dispensed with.

DESCRIPTION OF THE INVENTION

In search for an adequate substitute for the traditional sodium bicarbonate for treatment of acidosis, it has now been surprisingly found that amino- reduced sugar derivatives may be effective in the treatment of various pathological hyperacidity conditions.

In the course of developing the preparations of the present invention, it has also been found that pharmaceutical preparations comprising as active ingredient said amino-reduced sugar derivatives are also effective and advantageous in the treatment of gastric hyper-acidity.

It has also been found that oligomers of the amino-reduced sugar derivates, which are various poly-ethers can also be effectively used as antacid agents and are useful, in particular, as active ingredients in preparations for treatment of gastric hyper-acidity conditions.

The invention therefore relates to pharmaceutical preparations for treatment of hyperacidity of body fluids,

such as acidosis and gastric hyper-acidity comprising as active ingredient a compound of the general formula:

$$R^3-CH_2-(CHOH)_3-\overset{R^2}{C}H-CH_2R^1 \quad (I)$$

wherein each of $R^1$, $R^2$ and $R^3$ can represent a hydroxyl group or an amino group, optionally substituted by lower alkyl, at least one of $R^1$, $R^2$ and $R^3$ being said amino group, optionally substituted by lower alkyl, $R^1$ or $R^2$ and $R^3$ cannot simultaneously represent an amino group; or enantiomers or diastereoisomers thereof; or oligomers thereof; and pharmaceutically acceptable salts thereof optionally further comprising pharmaceutically acceptable additives in a suitable carrier or diluent.

Sugar can penetrate cell membranes only through specific carrier systems synethsized by the cell. It is well established that some reduced derivatives of sugars, like sorbitol or mannitol (as well as some poly-sugars) do not penetrate cells and if administered intravenously are confined to the extra-cellular space.

The amino derivatives of the reduced sugars are well known compounds. Some of them serve as intermediate compounds in the chemical industry. These compounds or N-methylated derivatives thereof are also known as inert additives to radiological contrast media, as described, for example, in Israel Patent No. 41556 (1973).

The amino derivatives of the impermeant reduced sugars are characterized by kidney clearances which are equal to the glomerular filtration rate which means that they would be execreted at a relatively high rate. Since these amino derivatives will bind hydrogen ions at a 1:1 molar ratio, the overall result will be enhancement of hydrogen ion excretion by the kidneys.

The compounds of formula I are very soluble in water, most of them are readily available and can be stored in various concentrations or even supplied in a dried form, for solubilization before use.

The pharmaceutical preparations of the invention may be used to combat acidosis in conjunction with or as substitutes for sodium bicarbonate in the pathological conditions described above.

In addition pharmaceutical preparations according to the present invention, comprising such additives as glucose and physiologically acceptable volume expanders like destran may be used as "survival solutions" in cases of blood loss as described above. As mentioned above, the kidney clearances of amino- reduced sugars employed in the preparations of the present invention are very good. Therefore, the "survival solutions" comprising glucose as energy source, may be effectively used for relatively long transient treatment of haemorrhaging patients, maintaining a more or less anaerobic metabolism, at least until it is possible to transfuse blood or even until blood is regentrated. Since sodium bicarbonate kidney clearance is low, solutions comprising sodium bicarbonate and glucose have not been used for these emergency conditions.

The pharmaceutical preparations of the invention have advantages over traditional bicarbonate physiological solutions in that sodium-free preparations are preferable for use with hypertensive elderly patients and wherever volume expansions is not desirable. The active ingredients of the present preparations, namely the compounds of formula I, are readily excreted through the kidneys, carrying hydrogen ions with them. Thus a constant slow infusion of the compounds of formula I can give a mild long-term antiacidic effect suitable especially for intensive-care units patients where a constant anti-acidotic treatment is advised. The preparations of the present invention may be preferable to sodium bicarbonate in first-aid resuscitation procedures where rapid alkalinization is advocated, since a rapid increase in sodium ion load and the increase in plasma $P_{CO_2}$ can have an adverse effect on heart performance and/or other vital functions (3,4).

The preparations of the present invention may also be repeatedly used to prevent nephrolithiasis when caused by uric acid (e.g. gout). The continued administration of urine pH and help the dissolution of uric acid stones by forming the corresponding uracyl-hydronium salt.

The preparations of the present invention may also be used as diagnostic preparations for evaluation of kidney function.

According to the second aspect of the invention, preparations comprising as active ingredient compounds of formula I or obligomers thereof, can be used as effective antacid agents for the treatment of gastric hyper-acidity.

The preparations of the present invention as gastric antacid agents are advantageous to the above known preparations since they do not contain metal ions such as magnesium, aluminum or calcium which may cause acid rebound. Oligomers of the compounds of formula I, as active ingredients in these gastric antacid preparations, may be preferable to the monomers since these relatively larger compounds will not penetrate the cell membranes. Thus, while the monomers are metabolized by gastrointestinal bacteria (10) and therefore may interfere with normal intestinal flora, the oligomers will be inert in this respect, namely not available to gastrointestinal flora, thus affecting only the gastric lumen pH.

Preferred preparations according to the present invention are those in which the active ingredient is a compound of formula I in which $R^1$ represents an amino group, optionally substituted by lower alkyl, and $R^2$

and $R^3$ both represent hydroxyl. These compounds are 1-amino-1-deoxy glucitol and N-alkylated derivatives thereof.

Of these preferred compounds, 1-deoxy-1-(methylamino)-gluicitol is particularly preferred (The common D-isomer is also known as N-methylglucamine).

The 1,6-di-amino derivatives, namely compounds of formula I in which $R^1$ and $R^3$ both represent an amino group, which may be optionally substituted by lower alkyl, and $R^2$ represents hydroxyl, may also be advantageous. The compounds may be prepared by known methods, for example, by treatment of the commercially available 1,6-dibromomannitol with two equivalents of sodamide or ammonia.

Various isomers (e.g. diastereoisomers and enantiomers) of compounds of formula I can be prepared. Thus, heating of N-methylglucamine beyond its melting point (160° C) for 5 minutes produces a diastereoisomer thereof. Mass spectroscopy analysis shows a quantitatively different fragmentation pattern than that of N-methylglucamine. The buffer properties of the diastereoisomer are similar to those of N-methylglucamine.

Oligomers of the compounds of formula I may also be prepared, for example by heating N-methylglucamine for 30 minutes at 170° C. The obligomers are thought to be made up of from 2 to 10 monomer units. They are practically oligoethers containing amino (optionally substituted) groups. The oligomers retain their buffer capacity per unit weight of the compound in comparison with the monomer.

For use as "survival solutions", discussed above, pharmaceutical aqueous solutions comprising glucose, 1-deoxy-1-(methylamino)-D-glucitol (NMG), dextran, electrolytes at physiological pH may be used. The aqueous solutions may contain, for example, 100 mM glucose, 5-50 mM NMG, 0,05 to 1 mM dextran (high concentrations being required at initiate stages, lower concentrations for maintenance stages), NaCl to isosmolarity, pH 7,2-7,4.

EXAMPLE I

In vitro use of preparations of the invention as antiacidotic agents.

An isotonic solution (300 mM) of 1-deoxy-1-(methylamino)-D-glucitol (NMG) was added to cat-blood to final concentration of 5mM and caused an increase in pH and a decrease of the partial pressure of $CO_2$, $P_{CO_2}$ .

No hemolysis was observed. Results are given in the appended Figure I.

EXAMPLE II

In vivo use of preparations of the invention as antiacidotic agents.

In vivo rapid (about 15 sec.) administration of 1cc/Kg of 300 mM isotonic solution of 1-deoxy-1(methylamino)-D-glucitol (NMG) to artificially ventilated cats caused immediate elevation of blood pH (from about 7.4 to 7.9), accompanied by a clear decrease in $P_{CO_2}$ .

The recovery of the pH to the normal value follows roughly an exponential course with a time constant of 1-3 minutes. Parallel increase in urine pH was observed.

There was no significant effect on blood pressure, heart rate or blood electrolytes concentration, see Table I. In spontaneoulsy breathing animals there was no significant effect on respiration. No gross deleterious effects were observed in the vein through which the solution was administered. Results are given in the appended Figure II.

EXAMPLE 3

Rat gastric juice was titrated with aqueous solutions of sodium bicarbonate (150 mM), 1-deoxy-1(methylamino)-D-gluicitol (NMG) (150 mM) and polymerized NMG (NMGP) (20 mg/cc). Results are given in the appended Figure III. The results clearly show that preparations comprising NMG and NMGP are more potent than sodium bicarbonate.

TABLE I

PLASMA CONCENTRATION OF VARIOUS COMPONENTS BEFORE, 1, 30, AND 120 MINUTES AFTER I.V. ADMINISTRATION OF NMG (0.3 mmol/kg) TO CAT.

| | Before | After | | |
|---|---|---|---|---|
| | | 1 min. | 30 min. | 120 min. |
| Sodium (mM) | 142 | 145 | 144 | 147 |
| Potassium (mM) | 4.2 | 4.2 | 4.2 | 4.2 |
| Chloride (mM) | 113 | 112 | 113 | 116 |
| Urea (mM) | 7 | 6.9 | 6.8 | 6.6 |


REFERENCES

1. Mudge G.H. Drugs affecting renal function and electrolyte metabolism. In: The Pharmacological Basis of therapeutics. (Goodman A.G., Gilman A. eds) pp. 885-915 VI Edition, Macmillan Publishing CO., Inc. New-York, (1980).

2. Corbet A.J., Adams J.M., Kenny J.D. Controlled trial of bicarbonate therapy in high-risk premature newborn infants. J. Pediatr. 91, 771 (1977).

3. Bishop R.L. and Weisfeld M.L. Sodium bicarbonate administration during cardiac arrest. Effect on arterial pH, $P_{CO2}$ and osmolality JAMA 234, 506 (1976)

4. Berenyi K.J., Wolk M. and Killip T. Crebospinal fluid acidosis complicating therapy of experimental cariopulmonary arrest. Circulation 52, 319 (1975).

5. Blaustein M.P. Sodium transport and hypertension (Editorial). Hypertension 6, 445-453 (1984).

6. Wilkund L. and Sahlin K. Induction and treatment of metabolic acidosis: A study of pH changes in porcine skeletal muscle and cerebrospinal fluid. Critical Care Medicine, 13, 109-113 (1985).

7. Strauss J. Tris (hydroxymethyl) amino-methane (THAM): A pediatric evaluation. Pediatrics 41, 667 (1968).

8. Harvey S.C. Gastric antacids and digestants. In The pharmacological basis of therapeutics. VI Edition, (Goodman L. S. and Gilman A. eds) pp. 988-1001 (Macmillan Publishing Co. Inc.) (1980).

9. Feurle G., Effect of rising intragastric pH induced by several antacids on serum gastrin concentrations in deudenal ulcer patients and in control group. Gastroenterology 68, 1-7 (1975).

10. Ryan A.J. Weitzel P.F. and Benness, G.T. The metabolism and excretion of N-methyl Glucamine in the rat. Life Sci. 19, 1925-1927 (1976).


**Claims**

1. Pharmaceutical preparations for the treatment of hyperacidity in body fluids comprising as active ingredient a compound of the formula:

$$R^3-CH_2(CHOH)_3-\overset{R^2}{CH}-CH_2R^1 \qquad (I)$$

wherein each of $R^1$, $R^2$ and $R^3$ represents a hydroxyl group or an amino group optionally substituted by lower alkyl, at least one of $R^1$, $R^2$ and $R^3$ being said amino group, optionally substituted by lower alkyl, provided that $R^2$ and $R^1$ or $R^2$ and $R^3$ cannot simultaneously represent an amino group; or enantiomers or diastereoisomers thereof; or oligomers thereof; and pharmaceutically acceptable salts thereof, optionally comprising pharmaceutically acceptable additives, in a suitable carrier or diluent.

2. A pharmaceutical preparation according to claim 1 in which the active ingredient in a compound of formula I in which $R^1$ is an amino group, optionally substituted by lower alkyl and $R^2$ and $R^3$ are hydroxyl.

3. A pharmaceutical preparation according to claim 2 in which the active ingredient is 1-amino-1-deoxy-glucitol.

4. A pharmaceutical preparation according to claim 2 in which the active ingredient is 1-deoxy-1-(methylamino)-glucitol.

5. Pharmaceutical preparations according to any of claims 1 to 4 suitable for intravenous administration, for treatment of hyperacidemia.

6. A pharmaceutical preparation according to claim 1 in which the active ingredient is a compound of formula I wherein $R^2$ is an amino group, optionally substituted by lower alkyl, and $R^1$ and $R^3$ are each hydroxyl.

7. A pharmaceutical preparation according to claim 1 in which the active ingredient is a compound of formula I wherein $R^3$ is an amino group, optionally substituted by lower alkyl and $R^1$ and $R^2$ are each hydroxyl.

8. A pharmaceutical preparation according to claim 1 in which the active ingredient is a compound of formula I wherein $R^1$ and $R^3$ are each an amino group, optionally substituted by lower alkyl, and $R^2$ is hydroxyl.

9. A pharmaceutical preparation according to claim 8 wherein the active ingredient is 1,6-deoxy -1,6-diamino-glucitol.

10. Pharmaceutical preparations for treatment of gastric hyperacidity according to any one of claims 1 to 4 and 6 to 9, suitable for per os administration.

11. Pharmaceutical preparations for treatment of gastric hyperacidity according to any one of claims 1 to 4 and 6 to 9 wherein the active ingredient is an oligomer of the compound of formula I, suitable for per os administration.

12. Pharmaceutical preparations according to claim 11 wherein the said oligomer is made up of from 2 to 10 monomer units of formula I.

13. A pharmaceutical preparation according to any one of claims 1 to 4 in which said additives are glucose, pharmaceutically acceptable volume expanding substances and electrolytes, having a physiological pH, suitable for intravenous administration, for treatment of blood-loss conditions.

14. A pharmaceutical preparation according to claim 13 comprising 1-amino-1-deoxy-glucitol or pharmaceutically acceptable salts thereof, glucose, dextran and sodium chloride.

0275998

FIGURE I

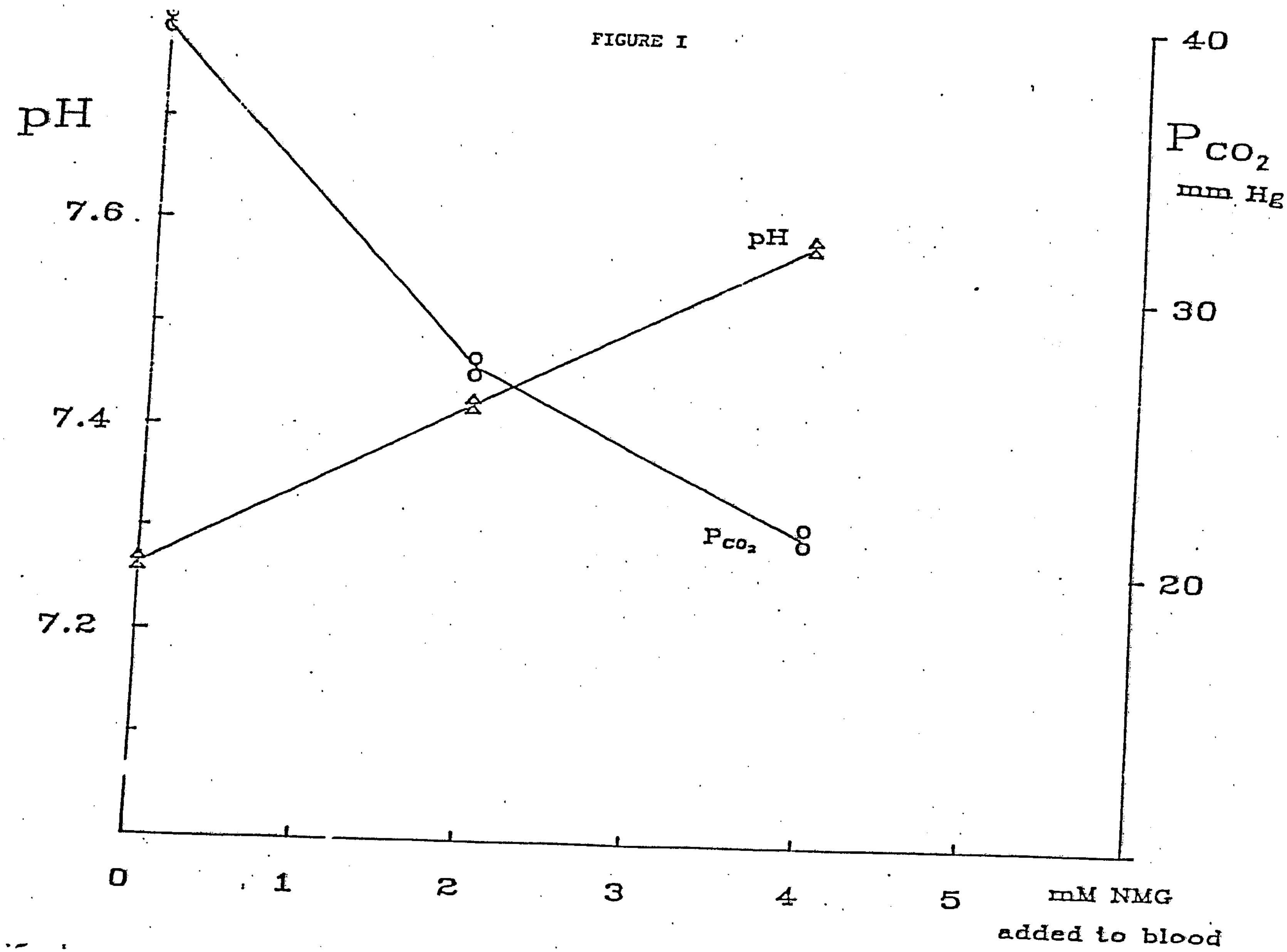

0275998

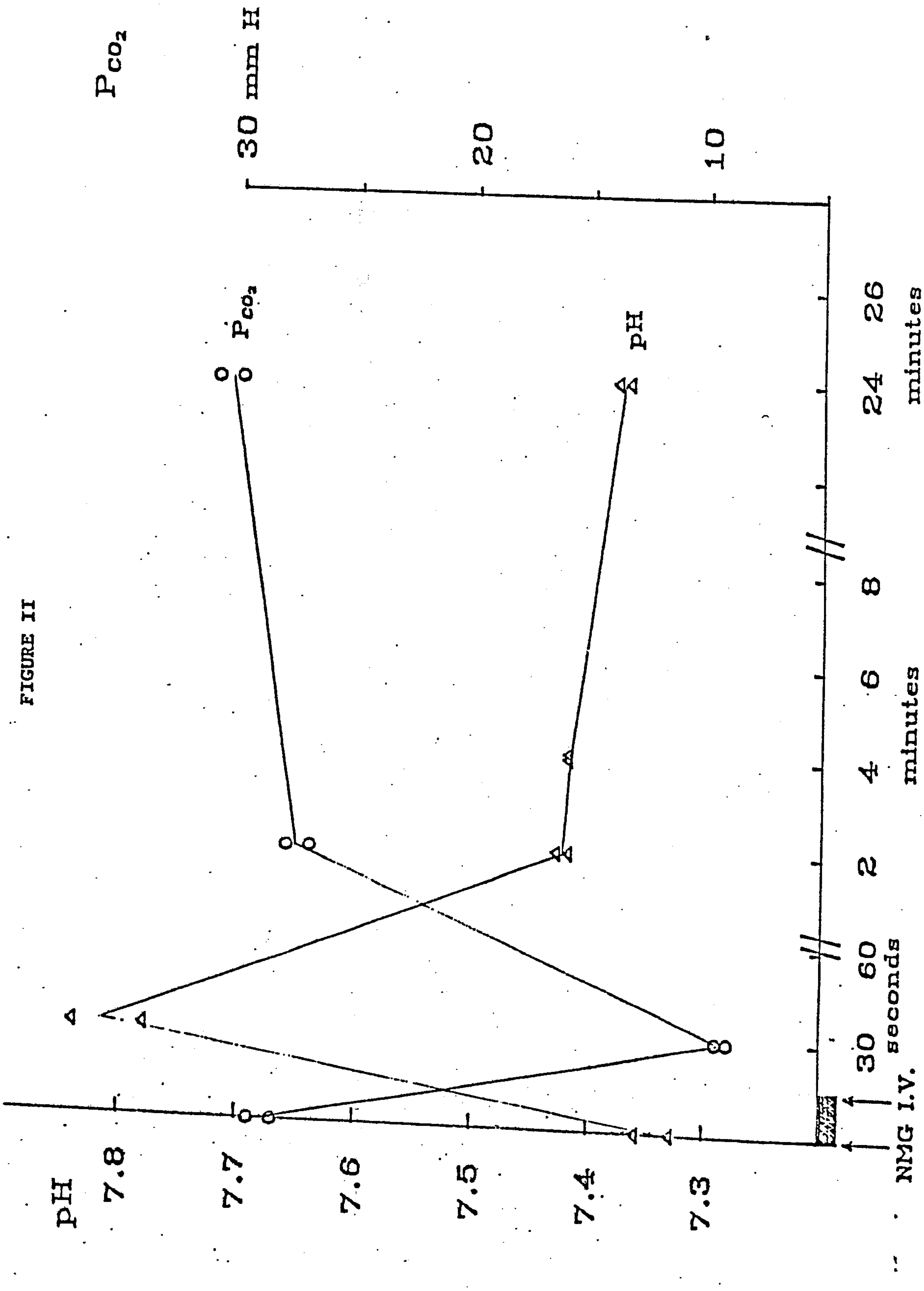


FIGURE II

FIGURE III

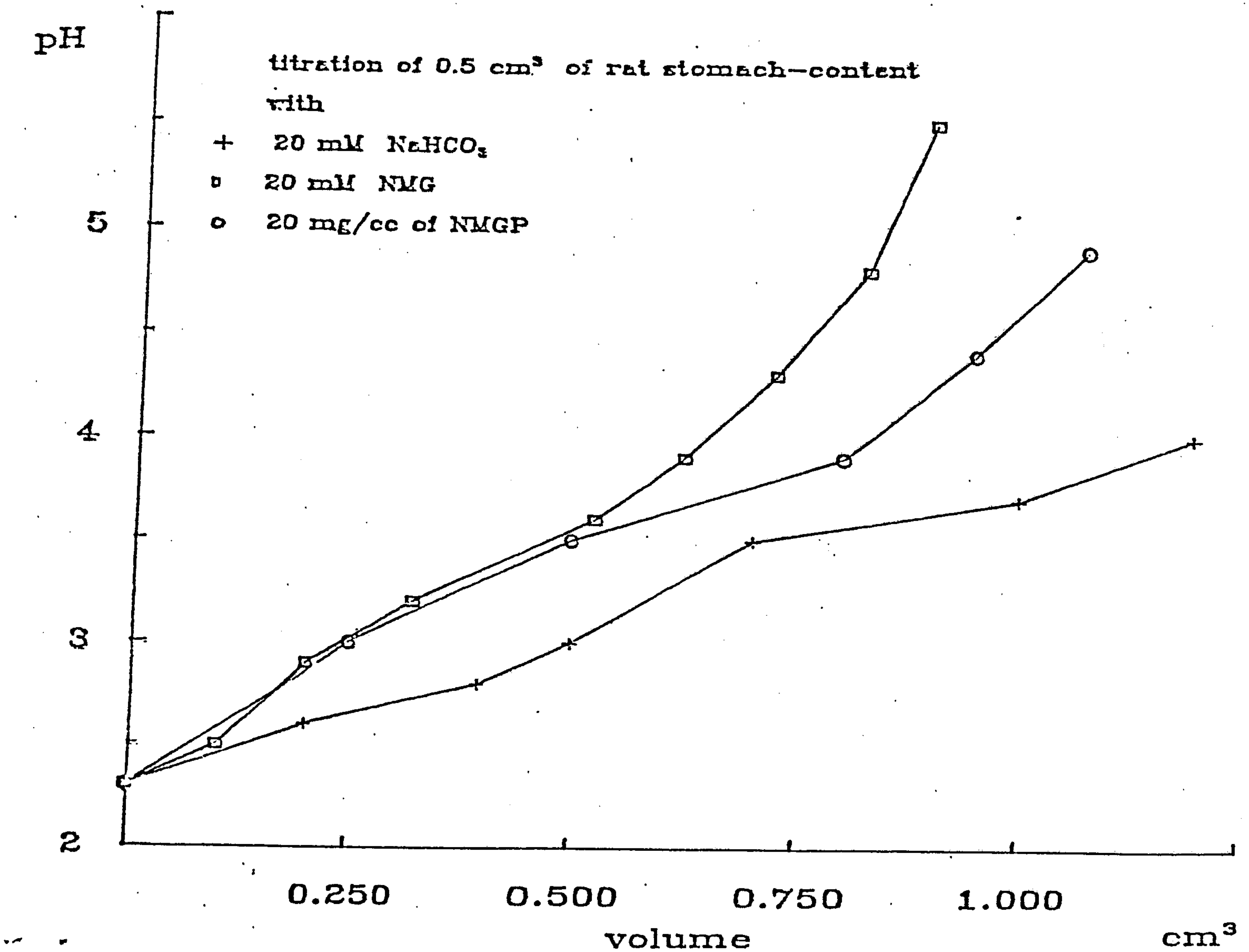
pH
5
4
3
2
titration of 0.5 cm³ of rat stomach-content
with
+ 20 mM NaHCO₃
▫ 20 mM NMG
o 20 mg/cc of NMGP
0.250
0.500
0.750
1.000
volume
cm³